# EUROPÄISCHE PATENTANMELDUNG

(11) **EP 1 619 187 A1**
(43) Veröffentlichungstag der Anmeldung: **25.01.2006**
(21) Anmeldenummer: 04017424.5
(22) Anmeldetag: 23.07.2004
(51) Int. Cl.: C07D 243/04

(54) **Verfahren zur Herstellung von 3-(4-Piperidinyl)- 2,3,4,5-tetrahydro-1,3-benzodiazepin-2(1H)-on**

(71) Anmelder: Boehringer Ingelheim Pharma GmbH & Co. KG, 55216 Ingelheim am Rhein (DE)
(72) Erfinder: Die Erfindernennung liegt noch nicht vor

(57) **Zusammenfassung**

Gegenstand der vorliegenden Erfindung ist ein Verfahren zur Herstellung der Verbindung 3-(4-Piperidinyl)-2,3,4,5-tetrahydro-1,3-benzodiazepin-2(1*H*)-on der Formel 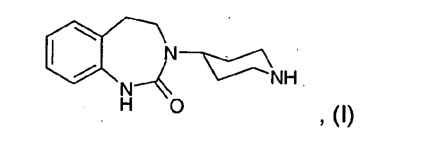 welche als Strukturelement in CGRP-Antagonisten, die sich vor allem zur oralen Therapie von Migräne eignen, zu finden ist.

## Beschreibung

Gegenstand der vorliegenden Erfindung ist ein Verfahren zur Herstellung der Verbindung 3-(4-Piperidinyl)-2,3,4,5-tetrahydro-1,3-benzodiazepin-2(1*H*)-on der Formel 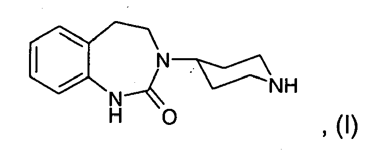
welche als Strukturelement in CGRP-Antagonisten, die sich vor allem zur oralen Therapie von Migräne eignen, zu finden ist.
Beispiele für Verbindungen mit CGRP-antagonistischen Eigenschaften, die als Strukturelement die Verbindung der Formel (I) enthalten, werden in den internationalen Patentanmeldungen PCT/EP97/04862, PCT/EP00/02004, PCT/EP00/13236, PCT/EP03/02417, PCT/EP03/11762 und PCT/EP03/11763 beschrieben.

Als Ausgangsstoff für die Verbindung der Formel I kann 2-Nitrophenylessigsäure verwendet werden. Sie wird in einem ersten Schritt mit einer äquimolaren Lösung aus 4-Amino-*N*-phenylmethylpiperidin in Gegenwart von mindestens einem Äquivalent, vorzugsweise 1.1 bis 1.5 Äquivalenten, besonders bevorzugt 1.1 Äquivalenten, Kondensationsmittel wie Carbonyldiimidazol, Carbonylditriazol, n-Propanphosphonsäureanhydrid, Dicyclohexylcarbodiimid, Thionylchlorid, TBTU O-(Benzotriazol-1-yl)-N,N,N',N'-tetramethyluroniumtetrafluroborat oder 1-Ethyl-3-(3'-dimethylaminopropyl)-carbodiimid zum 2-Nitro-*N* [1-(phenylmethyl)-4-piperidinyl]-phenylacetamid umgesetzt. Geeignete Lösungsmittel sind polare aprotische Lösungsmittel wie Tetrahydrofuran, Dimethoxyethan, Toluol, Dimethylformamid oder N-Methylpyrrolidinon. Das Produkt kann z.B. durch Verdünnen mit Wasser zum Auskristallisieren gebracht und durch Filtration oder Zentrifugation und Trocknung aufgearbeitet werden.
Im folgenden Schlüsselschritt wird das 2-Nitro-*N*-[1-(phenylmethyl)-4-piperidinyl]-phenylacetamid zuerst in einem polaren aprotischen organischen Lösungsmittel, wie beispielsweise Tetrahydrofuran oder Dimethoxyethan, gelöst und die Carbonylgruppe durch Zugabe von mindestens einem Äquivalent, vorzugsweise 2.0 bis 4.0 Äquivalenten, eines Reduktionsmittels in eine Methylengruppe übergeführt. Als Reduktionsmittel kommen beispielsweise Boran, Lithium- oder Natriumborhydrid in Frage, gegebenenfalls unter Zusatz von mindestens 0.5 Äquivalenten, vorzugsweise 2.0 bis 4.0 Äquivalenten, einer Lewissäure, einer Säure oder eines Halogens, beispielsweise unter Zusatz von Schwefelsäure, Chlortrimethylsilan oder lod. Die Reduktion kann bei Temperaturen von 20 bis 70°C, vorzugsweise bei 60 bis 70°C, durchgeführt werden.
Anschließend wird die Nitrogruppe des so erhaltenen Zwischenprodukts *N*-[2-(2-Nitrophenyl)ethyl]-1-(phenylmethyl)-4-amino-piperidin in Gegenwart eines Raney-Nickel Katalysators hydriert. Für die Hydrierung wird der Ausgangsstoff in Dimethylformamid vorgelegt und der Katalysator als wässrige Suspension zugegeben. Vorteilhafte Bedingungen für die Hydrierung sind Temperaturen von 20 bis 60°C und ein Wasserstoffüberdruck von maximal 3 bar. Nach dem Abfiltrieren des Katalysators kann das Hydrierungsprodükt durch Abdestillieren des Lösungsmittels aufkonzentriert werden. Danach erfolgt die Cyclisierung, indem das so erhaltene Rohprodukt in eine Suspension von mindestens einem Äquivalent, vorzugsweise 1.1 bis 1.75 Äquivalenten, besonders bevorzugt 1.1 Äquivalenten, eines Kondensationsmittels, wie beispielsweise Carbonyldiihiidazol oder Carbonylditriazol, zugegeben wird. Geeignete Lösungsmittel sind polare aprotische Lösungsmittel wie Tetrahydrofuran, Ethylacetat, Dimethylformamid oder *N*-Methylpyrrolidinon. Das so erhaltene 3-[1-(Phenylmethyl)-4-piperidinyl]-2,3,4,5-tetrahydro-1,3-benzodiazepin-2(1*H*)-on kann z.B. durch Verdünnen mit Wasser und Alkohol, beispielsweise Methanol, Ethanol oder Isopropanol, vorzugsweise Methanol, ausgefällt und durch Filtration oder Zentrifugation und Trocknung aufgearbeitet werden.
Gegebenenfalls kann das Zwischenprodukt *N*-[2-(2-Nitrophenyl)ethyl]-1-(phenylmethyl)-4-amino-piperidin in Form eines Salzes durch Zugabe von mindestens 2 Äquivalenten einer wässrigen Lösung einer starken Säure ausgefällt und isoliert werden. Beispiele für geeignete Säuren sind Salzsäure, Bromwasserstoffsäure oder Schwefelsäure, insbesondere Salzsäure, wobei das Dihydrochlorid erhalten wird. Dazu wird das Reaktionsgemisch auf Raumtemperatur abgekühlt und mit einem Alkohol, wie beispielsweise Methanol, Ethanol oder Isopropanol, vorzugsweise Methanol, versetzt. Nach Zugabe eines Überschusses der wässrigen Säure wird erneut zum Rückfluss erhitzt und anschließend wiederum abgekühlt. Das Reaktionsgemisch wird mit einer wässrigen Lösung eines Base, beispielsweise Lithiumhydroxid, Natriumhydroxid, Ammoniak oder Kaliumhydroxid, alkalisch gestellt und nach Phasentrennung wird die organische Phase mit einem Überschuss der wässrigen Säure versetzt, wobei das gewünschte Salz auskristallisiert. Das Produkt kann dann durch Filtration oder Zentrifugation und Trocknung aufgearbeitet werden.

In einem dritten Schritt wird die Benzylschutzgruppe des 3-[1-(Phenylmethyl)-4-piperidinyl]-2,3,4,5-tetrahydro-1,3-benzodiazepin-2(1*H*)-ons abgespalten. Dazu wird der Ausgangsstoff in einem polaren Lösungsmittel, wie beispielsweise Methanol, Ethanol, Wasser, Aceton, Tetrahydrofuran, Dimethylformamid oder Propanol, gelöst, und in einem Druckreaktor hydriert. Als Hydrierungsmittel können beispielsweise Pd/C oder Pd(OH)₂ verwendet werden. Vorteilhafte Bedingungen für die Hydrierung sind Temperaturen von 40 bis 80°C und ein Wasserstoffüberdruck von maximal 3 bar. Nach dem Abfiltrieren des Katalysators kann das Hydrierungsprodukt 3-(4-Piperidinyl)-2,3,4,5-tetrahydro-1,3-benzodiazepin-2(1*H*)-on der Formel (I) durch Aufkonzentrierung des Lösungsmittels und anschließender Zugabe von Aceton oder Wasser kristallisiert, abfiltriert und getrocknet werden.

### Experimenteller Teil

### Beispiel 1: 2-Nitro-N-[1-(phenylmethyl)-4-piperidinyl]-phenylacetamic

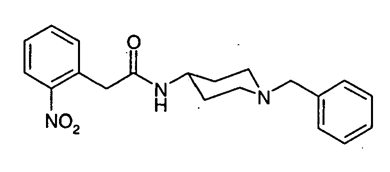

11.81 kg (72.87 mol, 1.1 eq) 1,1-Carbonyldiimidazol (CDI) werden bei 20°C vorgelegt und 18 L Tetrahydrofuran zugegeben. Anschließend werden 12.00 kg (66.24 mol, 1.0 eq) 2-Nitrophenylessigsäure, gelöst in 24 L Tetrahydrofuran, innerhalb von 15 Minuten zugegeben. Das Zulaufgefäß wird mit 9 L Tetrahydrofuran gespült und das Reaktionsgemisch 30 Minuten gerührt (Gasentwicklung: CO₂). Anschließend wird zweimal ein Vakuum von 300 mbar angelegt, um überschüssiges CO₂ zu entfernen.

Zur Lösung werden bei 20°C 12.61 kg (66.24 mol, 1.0 eq) 4-Amino-*N*-phenylmethylpiperidin in 6 L Tetrahydrofuran geben (exotherm). Nach der Zugabe wird das Reaktionsgemisch noch 2 Stunden bei 20°C gerührt. Danach werden 144 L Wasser zugegeben, wobei die Lösung nach Zugabe von ¼ der Wassermenge mit 2-Nitro-*N*-[1-(phenylmethyl)-4-piperidinyl]-phenylacetamid angeimpft wird. Die erhaltene Suspension wird auf 0 bis 5°C abgekühlt und zur Vervollständigung der Kristallisation eine weitere Stunde gerührt. Anschließend wird das Produkt abzentrifugiert, mit einem kalten Gemisch aus 160 L Wasser und 9 L Tetrahydrofuran gewaschen und unter Inertisierung im Trockenschrank bei 45°C getrocknet.
Ausbeute: 18.21 kg (77.8% der Theorie)
Chemische Reinheit laut HPLC: 99.8 %

### Beispiel 2: N-[2-(2-Nitrophenyl)ethyl]-1-(phenylmethyl)-4-amino-piperidin-dihydrochlorid

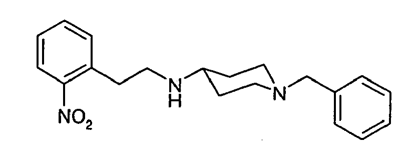

10.00 kg (28.29 mol, 1.0 eq) 2-Nitro-*N*-[1-(phenylmethyl)-4-piperidinyl]-phenylacetamid aus Beispiel 1 werden in 60 L Tetrahydrofuran vorgelegt und auf 60°C erhitzt. 11.06 kg (101.84 mol, 3.6 eq) Chlortrimethylsilan und 14.79 kg (67.90 mol, 2.4 eq) einer 10%-igen Lithiumborhydrid-Lösung in THF werden bei 60 bis 65°C (Gasentwicklung) innerhalb von 15 Minuten zudosiert. Das Reaktionsgemisch wird 4 Stunden nachgerührt, anschließend auf 20°C abgekühlt und mit 7 L Methanol versetzt. Nach Zugabe von 14.80 kg (121.65 mol, 4.3 eq) 30%-iger technischer Salzsäure wird das Reaktionsgemisch 2.5 Stunden unter Rückfluss gerührt, auf 20°C abgekühlt und mit 8.38 kg (104.67 mol, 3.7 eq) 50%-iger technischer Natriumhydroxid-Lösung wird ein pH-Wert von 9.2 eingestellt. Die Wasserphase wird abgetrennt, die organische Phase mit 6.89 kg (56.58 mol, 2.0 eq) 30%-iger technischer Salzsäure versetzt (pH 1.5) und 1 Stunde unter Rückfluss gerührt. Die erhaltene Suspension wird innerhalb von 3 Stunden auf 0°C abgekühlt. Zur Vervollständigung der Kristallisation wird 1 Stunde bei 0°C gerührt. Anschließend wird das Produkt abzentrifugiert, mit 20 L Tetrahydrofuran gewaschen und unter Inertisierung im Trockenschrank bei 50°C getrocknet.
Ausbeute: 7.78 kg (66.7% der Theorie)
Chemische Reinheit laut HPLC: 99.3 %

### Beispiel 3: 3-[1-(Phenylmethyl)-4-piperidinyl]-2,3,4,5-tetrahydro-1,3-benzodiazepin-2(1H)-on

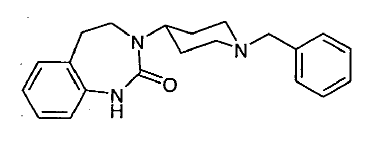

10.00 kg (24.25 mol, 1.0 eq) *N*-[2-(2-Nitrophenyl)ethyl]-1-(phenylmethyl)-4-amino-piperidin-dihydrochlorid aus Beispiel 2 werden in je 30 L Toluol und Wasser vorgelegt. 5.82 kg (72.75 mol, 3.0 eq) 50%-ige technische Natriumhydroxid-Lösung werden zudosiert und das zweiphasige Gemisch 1 Stunde bei 50°C gerührt. Die Wasserphase wird abgetrennt, die organische Phase mit 12 L Wasser gewaschen und anschließend im Vakuum eingeengt. Der Rückstand wird mit 50 L Methanol versetzt. Ein Teil des eingesetzten Methanols wird abdestilliert. Die verbleibende Lösung wird in Gegenwart von 860 g Raney Nickel bei 50°C hydriert. Der Katalysator wird abfiltriert und mit 16 L Methanol gewaschen. Das Lösungsmittel wird abdestilliert und der Rückstand mit 30 L Tetrahydrofuran versetzt. Die Hälfte des eingesetzten Tetrahydrofurans wird abdestilliert und die verbleibende Lösung bei 20°C zu einer Suspension aus 6.88 kg (42.44 mol, 1.75 eq) 1,1-Carbonyldiimidazol in 15 L Tetrahydrofuran innerhalb von 1.5 Stunden zudosiert. Es wird 1 Stunde bei dieser Temperatur nachgerührt. Anschließend werden 20 L Wasser zugegeben, angeimpft und weitere 17 L Wasser zugegeben. Die erhaltene Suspension wird auf 0°C abgekühlt. Zur Vervollständigung der Kristallisation wird 1.5 Stunden bei 0°C gerührt. Anschließend wird das Produkt abzentrifugiert, mit 30 L Wasser gewaschen und unter Inertisierung im Trockenschrank bei 45°C getrocknet.
Ausbeute: 6.38 kg (78.4% der Theorie)
Chemische Reinheit laut HPLC: 98.5%

### Beispiel 4: 3-[1-(Phenylmethyl)-4-piperidinyl]-2,3,4,5-tetrahydro-1,3-benzodiazepin-2(1H)-on

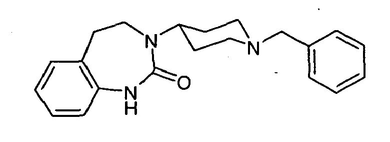

17.00 kg (48.10 mol, 1.0 eq) 2-Nitro-*N*-[1-(phenylmethyl)-4-piperidinyl]-phenylacetamid aus Beispiel 1 werden in 170 L Ethylenglykoldimethylether suspendiert und auf 0 bis 5 °C abgekühlt. Bei dieser Temperatur werden portionsweise 7.28 kg (192.40 mol, 4.0 eq) Natriumborhydrid und anschließend eine Lösung von 9.91 kg (101.01 mol, 2.1 eq) technische Schwefelsäure in 17 L Ethylenglykoldimethylether zugegeben. Nach beendeter Zugabe wird mit 6.8 L Ethylenglykoldimethylether gespült. Innerhalb von 1 Stunde wird das Reaktionsgemisch auf 70°C aufgeheizt und 4 Stunden bei dieser Temperatur gerührt.
Nach Abkühlen auf 55°C wird eine Lösung aus 29 L Wasser und 11.69 kg (96.20 mol, 2.0 eq) 30%-ige technische Salzsäure zudosiert. Nach beendeter Zugabe wird mit 5 L Wasser nachgespült. Bei 70°C wird noch 1.5 Stunden gerührt. Anschließend wird das Reaktionsgemisch auf 20°C abgekühlt, mit 30.0 kg (375.18 mol, 7.8 eq) 50%-iger technischer Natriumhydroxid-Lösung und 17 L Wasser versetzt. Nach der Phasentrennung wird die organische Phase im Vakuum bis zu einem Öl eingeengt und mit 43 L Dimethylformamid versetzt.
Die wie oben erhaltene Lösung wird aus dem Reaktor in einen Behälter abgelassen und in den Hydrierreaktor überführt. 1.35 kg Raney-Nickel KatalysatorSuspension, die zuvor dreimal mit je 3 L Dimethylformamid versetzt und abdekantiert wurde, werden in 3 L Dimethylformamid suspendiert und eingesaugt. Anschließend wird bei 3 bar und einer Innentemperatur von 50°C hydriert, bis keine Wasserstoffaufnahme mehr zu verzeichnen ist. Der Katalysator wird abfiltriert und mit 17 L Dimethylformamid gewaschen. Anschließend werden im Vakuum mindestens 46 L des Lösungsmittels abdestilliert. Liegt der Wassergehalt des Destillats nach dieser Menge nicht unter 1%, wird weiter destilliert, bis der gewünschte Wert erzielt ist.

8.58 kg (52.91 mol, 1.1 eq) 1,1-Carbonyldiimidazol werden vorgelegt und mit 32 L Dimethylformamid versetzt. Anschließend wird bei einer Temperatur von 20°C Hydrierlösung (aus vorangegangenem Reaktionsschritt) innerhalb von 2 Stunden zudosiert. Nach 30 Minuten Rührzeit wird auf Vollständigkeit der Umsetzung überprüft. Danach wird auf 50°C aufgeheizt und ein Gemisch aus 34 L Methanol und 136 L Wasser innerhalb von 35 Minuten zudosiert. Zur Vervollständigung der Kristallisation werden bei 50°C noch 34 L Wasser zugegeben und die Suspension innerhalb von 1 Stunde auf 10°C abgekühlt und 30 Minuten bei dieser Temperatur gerührt. Anschließend wird der Niederschlag abzentrifugiert, mit 85 L Wasser gewaschen und bei 50°C im Trockenschrank getrocknet.
Ausbeute: 12.73 kg (78.9% der Theorie)
Chemische Reinheit laut HPLC: 96.0%

### Beispiel 5: 3-(4-Piperidinyl)-2,3,4,5-tetrahydro-1,3-benzodiazepin-2(1H)-on

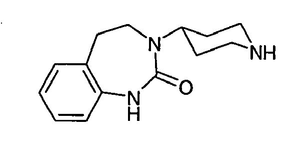

10.00 kg (29.81 mol, 1.0 eq) 3-[1-(Phenylmethyl)-4-piperidinyl]-2,3,4,5-tetrahydro-1,3-benzodiazepin-2(1*H*)-on aus Beispiel 3 werden in 100 L Methanol gelöst, mit 1.00 kg 10%-igem Pd/C versetzt und im Druckreaktor bei 70°C und 3 bar hydriert. Nach Beendigung der Wasserstoffaufnahme wird der Katalysator abfiltriert und mit 30 L Methanol gewaschen. Das Filtrat wird im Vakuum aufkonzentriert und der Rückstand in 100 L Aceton suspendiert. Danach wird zum Rückfluss erhitzt, die Suspension 15 Minuten unter Rückfluss gerührt und die Hälfte des Acetons bei Normaldruck abdestilliert. Nach beendeter Destillation wird auf 0°C abgekühlt und eine weitere Stunde gerührt. Das Produkt wird abgesaugt, mit 20 L Aceton gewaschen und bei 50°C getrocknet.
Ausbeute: 6.17 kg (84.3% der Theorie)
Chemische Reinheit laut HPLC: 99.8%

## Patentansprüche

1. Verfahren zur Herstellung der Verbindung der Formel 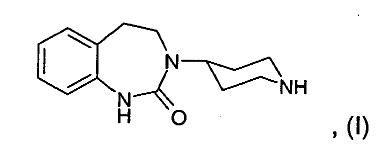
**dadurch gekennzeichnet, dass**
(a) 2-Nitrophenylessigsäure in Gegenwart von Kondensationsmitteln mit 4-Amino-N-phenylmethylpiperidin umgesetzt wird;
(b) die Carbonylgruppe des erhaltenen 2-Nitro-*N*-[1-(phenylmethyl)-4-piperidinyl]-phenylacetamids durch Zugabe eines Reduktionsmittels- in eine Methylengruppe übergeführt wird;
(c) das erhaltene Zwischenprodukt *N*-[2-(2-Nitrophenyl)ethyl]-1-(phenylmethyl)-4-amino-piperidin nach Reduktion der Nitrogruppe in Gegenwart eines Raney-Nickel Katalysators durch Zugabe von Kondensationsmitteln zu 3-[1-(Phenylmethyl)-4-piperidinyl]-2,3,4,5-tetrahydro-1,3-benzodiazepin-2(1*H*)-on cyclisiert wird und
(d) das 3-[1-(Phenylmethyl)-4-piperidinyl]-2,3,4,5-tetrahydro-1,3-benzodiazepin-2(1*H*)-on durch Abspalten der Benzylschutzgruppe in die Verbindung der Formel (I) übergeführt wird.

2. Verfahren gemäß Anspruch 1, **dadurch gekennzeichnet, dass** in Schritt (a) das 4-Amino-*N*-phenylmethylpiperidin als Lösung zugegeben und das bei der Reaktion erhaltene Produkt durch Verdünnen mit Wasser auskristallisiert wird.

3. Verfahren gemäß Anspruch 1, **dadurch gekennzeichnet, dass** in Schritt (b) das 2-Nitro-*N*-[1-(phenylmethyl)-4-piperidinyl]-phenylacetamid in einem polaren aprotischen organischen Lösungsmittel gelöst wird und die Reduktion bei Temperaturen von 20 bis 70°C, gegebenenfalls unter Zusatz einer Lewissäure, einer Säure oder eines Halogens, durchgeführt wird.

4. Verfahren gemäß Anspruch 1, **dadurch gekennzeichnet, dass** die Ausgangsverbindung in Schritt (c) in Dimethylformamid vorgelegt, der Katalysator als wässrige Suspension zugegeben und bei Temperaturen von 20 bis 60°C und einem Wasserstoffüberdruck von maximal 3 bar hydriert wird.

5. Verfahren gemäß Anspruch 1, **dadurch gekennzeichnet, dass** der Ausgangsstoff in Schritt (d) in einem polaren Lösungsmittel gelöst und nach Zugabe eines Hydrierungsmittels in einem Druckreaktor bei Temperaturen von 40 bis 80°C und einem Wasserstoffüberdruck von maximal 3 bar hydriert wird.

6. Verfahren gemäß Anspruch 1, **dadurch gekennzeichnet, dass** das in Schritt (b) erhaltene Zwischenprodukt *N*-[2-(2-Nitrophenyl)ethyl]-1-(phenylmethyl)-4-amino-piperidin in Form eines Salzes durch Zugabe einer wässrigen Lösung einer starken Säure ausgefällt und isoliert wird.

7. Verfahren gemäß Anspruch 4, **dadurch gekennzeichnet, dass** das erhaltene Produkt 3-[1-(Phenylmethyl)-4-piperidinyl]-2,3,4,5-tetrahydro-1,3-benzodiazepin-2(1*H*)-on durch Verdünnen mit Wasser und Alkohol ausgefällt wird.
